# EUROPÄISCHE PATENTANMELDUNG

(11) **EP 1 378 850 A1**
(43) Veröffentlichungstag der Anmeldung: **07.01.2004**
(21) Anmeldenummer: 02014702.1
(22) Anmeldetag: 03.07.2002
(51) Int. Cl.: G06F 19/00

(54) **Verfahren und System zur Unterstützung der Therapieplanung bei der Erstellung eines Trainingsprogrammes**

(71) Anmelder: SIEMENS AKTIENGESELLSCHAFT, 80333 München (DE); Dr. Hein GmbH, 90429 Nürnberg (DE)
(72) Erfinder: Abraham-Fuchs, Klaus, 91058 Erlangen (DE); Eisermann, Uwe, 91052 Erlangen (DE); Richter, Niels, 95349 Thurnau (DE); Setz, Robert, 91126 Rednitzhembach (DE)
(74) Vertreter: Berg, Peter, Dipl.-Ing.

(57) **Zusammenfassung**

Die vorliegende Erfindung betrifft ein Verfahren sowie ein System zur Unterstützung der Therapieplanung bei der Erstellung eines Trainingsprogrammes. Bei dem Verfahren werden ein Fähigkeitsprofil eines Patienten sowie eine erste (11) und zweite Datenbank (12) bereitgestellt. Die erste Datenbank (11) enthält mehrere Fertigkeiten und eine Zuordnung von Mindestvoraussetzungen an Fähigkeiten, die für die jeweiligen Fertigkeiten erforderlich sind. Die zweite Datenbank (12) enthält mehrere Fertigkeiten mit Expertenregeln über die Auswahl von Übungen und/oder zu behandelnden Fähigkeiten sowie deren Reihenfolge und/oder Gewichtung zur Erlangung der jeweiligen Fertigkeiten unter Berücksichtigung vorhandener Fähigkeiten bzw. Fähigkeitsdefizite. Von einer Datenverarbeitungsstation (10) werden für zumindest eine zu behandelnde Fertigkeit des Patienten automatisch das Fähigkeitsprofil durch Rückgriff auf die erste Datenbank (11) zur Ermittlung der vorhandenen Fähigkeiten bzw. Fähigkeitsdefizite ausgewertet und durch Rückgriff auf die zweite Datenbank (12) unter Berücksichtigung der Expertenregeln Übungen und/oder zu behandelnde Fähigkeiten ausgewählt und mit einer Information über die Gewichtung und/oder Reihenfolge bei der Durchführung eines Trainings ausgegeben. Das vorliegende Verfahren sowie das zugehörige System verringern den Zeitaufwand für den Arzt oder Therapeuten bei der Erstellung eines individuell an den Patienten angepassten Trainingsprogrammes.

## Beschreibung

Die vorliegende Erfindung betrifft ein Verfahren sowie ein System zur Unterstützung der Therapieplanung bei der Erstellung eines Trainingsprogrammes für die Rehabilitation eines Patienten.

Als Folge schwerer Erkrankungen wie bspw. Schlaganfall, Herzinfarkt oder Alzheimer' sche Krankheit oder als Folge schwerer operativer Eingriffe wie bspw. dem Einsatz von Gelenkimplantaten oder der Durchführung einer Amputation treten bei der Mehrzahl der Patienten unterschiedliche Defizite in der körperlichen und geistigen Leistungsfähigkeit auf. Diese Defizite sind in der Regel die Folge der Schwächung oder des kompletten Ausfalls einer Gehirnregion oder eines Muskels. Auch Kombinationen hiervon treten häufig auf. So kann z. B. eine Gehirnregion geschädigt sein, die für die Steuerung eines Muskels oder mehrerer Muskeln in funktionalen Ketten zuständig ist. Als Folge davon degenerieren die betroffenen Muskeln, so dass sie nicht mehr richtig eingesetzt werden können. Solche geistigen oder körperlichen Einschränkungen werden in der medizinischen Fachsprache als Fähigkeitsdefizite bezeichnet, die in verschiedene Fähigkeitsbereiche eingeteilt werden können. So ist unterscheidet eine bekannte Klassifizierung bspw.:
- motorische Fähigkeiten wie Kraft, Ausdauer, Beweglichkeit, Gleichgewicht, Reaktion, Orientierung, Differenzierung, Umstellung, Sprachmotorik;
- intellektuelle/kognitive Fähigkeiten wie Aufmerksamkeit, Gedächtnis, Planung, Sprachverständnis, Wortfindung, Sehen;
- organisch/physische Fähigkeiten wie bspw. die Verringerung der Organleistung;
- soziale Fähigkeiten wie bspw. die Kommunikations- und Partizipationsfähigkeit;
- emotionale Fähigkeiten wie bspw. die Fähigkeit zur Entwicklung des Selbstwertgefühls.
Einige Fähigkeiten erfordern auch ein Zusammenspiel motorischer und kognitiver Funktionen. So setzt bspw. die Tätigkeit des Treppensteigens Kraft und Gleichgewicht als motorische Fähigkeiten sowie Aufmerksamkeit und räumliche Wahrnehmung als kognitive Fähigkeiten voraus.

Sehr häufig tritt bei einem Patienten nicht ein einziges Defizit in einer Fähigkeitskategorie auf, sondern eine Kombination mehrerer Defizite in mehr oder weniger schwerer Ausprägung. Ziel einer therapeutischen Maßnahme, die meist im Rahmen eines Rehabilitationsprozesses durchgeführt wird, ist die Wiederherstellung der Fähigkeiten bzw. die weitest mögliche Verminderung der vorliegenden Defizite. Zu Beginn der rehabilitativen Maßnahme werden dabei in der Regel alle Fähigkeitsdefizite des Patienten anhand bekannter Verfahren zur Messung, Beobachtung und Befragung erfasst und ihr Ausmaß dokumentiert. Dieser Erfassungsprozess wird auch als Staging des Patienten bezeichnet. Je nach eingesetztem Messverfahren ist das Ergebnis dieses Staging-Prozesses quantitativ, bspw. in Prozent der Sehfähigkeit oder durch Angabe des Grades der Beweglichkeit des Oberarmes, oder qualitativ, bspw. durch eine Einstufung der Fähigkeitseinschränkung in schwer, mittelgradig oder leicht. Ein Beispiel eines etablierten Messverfahrens für das Staging zahlreicher neurologischer, kognitiver und psychischer Fähigkeiten ist die sog. Wiener Testbatterie der Firma Schuhfried.

Das Resultat dieser Eingangsuntersuchung ist im Idealfall ein fachübergreifender Fähigkeitsbericht, der sich in Form eines Fähigkeitsprofils darstellen lässt. Ein Fähigkeitsprofil wird in diesem Kontext als eine Liste aller relevanten Fähigkeiten und eine Zuordnung des Grades der Einschränkung dieser Fähigkeiten bei diesem Patienten zum Erhebungszeitpunkt definiert.

Neben dem Begriff Fähigkeiten wird in der medizinischen Fachsprache auch der Begriff Fertigkeit benutzt. Unter einer Fertigkeit wird im Kontext einer medizinischen Rehabilitationsmaßnahme eine komplexe Handlung verstanden, die aber in sich geschlossen und gegenüber anderen Handlungen abgrenzbar ist. Für eine Fertigkeit wird das Zusammenspiel mehrerer Fähigkeiten benötigt. Insbesondere bezieht sich der Begriff Fertigkeit im Kontext einer Rehabilitation auf Aktivitäten des täglichen Lebens (ADL: Activities of Daily Living), die Grundvoraussetzung für ein unabhängiges, selbständiges Leben sind. Beispiele für derartige Fertigkeiten sind das Einnehmen von Nahrung, das Anziehen, das Waschen, das Duschen, das Treppensteigen usw.. Die Ausführung solcher Fertigkeiten wird auch in standardisierten Fragebögen erfasst und als ADL-Index quantifiziert. Obwohl im Rahmen einer Rehabilitation in direkter Weise Fähigkeiten trainiert werden, ist das eigentliche Ziel die Wiedererlangung von Fertigkeiten.

Für die Verbesserung einer Fertigkeit eines Patienten ist in der Regel die Durchführung mehrerer Übungen zu unterschiedlichen, für die Fertigkeit relevanten Fähigkeiten erforderlich. Die Reihenfolge und Gewichtung dieser Übungen hängt u. a. von dem Grad der jeweiligen Fähigkeitsdefizite des Patienten ab. Der Arzt oder Therapeut muss bei der Erstellung eines Trainingsprogrammes diese Abhängigkeiten berücksichtigen und aufgrund seiner Erfahrung ein geeignetes Trainingsprogramm erstellen, das sich in der Regel aus mehreren Übungen zusammensetzt.

Die Aufgabe der vorliegenden Erfindung besteht darin, ein Verfahren sowie ein System zur Unterstützung der Therapieplanung bei der Erstellung eines Trainingsprogrammes anzugeben, das den Zeitaufwand für den zuständigen Arzt oder Therapeuten verringert und insbesondere die Erstellung eines Trainingsprogrammes für eine schnellstmögliche Erlangung der Fertigkeiten ermöglicht.

Die Aufgabe wird mit dem Verfahren sowie dem System der Patentansprüche 1 bzw. 12 gelöst. Vorteilhafte Ausgestaltungen des Verfahrens sowie des Systems sind Gegenstand der Unteransprüche oder lassen sich der nachfolgenden Beschreibung sowie den Ausführungsbeispielen entnehmen.

Bei dem vorliegenden Verfahren zur Unterstützung der Therapieplanung bei der Erstellung eines Trainingsprogrammes werden ein aktuelles Fähigkeitsprofil des Patienten sowie eine erste und eine zweite Datenbank bereitgestellt. Die erste Datenbank enthält mehrere Fertigkeiten sowie eine Zuordnung von Mindestvoraussetzungen an Fähigkeiten, die für die jeweilige Fertigkeit erforderlich sind. Die zweite Datenbank enthält mehrere Fertigkeiten mit Expertenregeln über die Auswahl von Übungen und/oder zu behandelnden Fähigkeiten sowie deren Reihenfolge und/oder Gewichtung zur Erlangung der jeweiligen Fertigkeiten unter Berücksichtigung vorhandener Fähigkeiten bzw. Fähigkeitsdefizite. Von einer Datenverarbeitungsstation wird für zumindest eine zu behandelnde Fertigkeit des Patienten automatisch das Fähigkeitsprofil des Patienten durch Rückgriff auf die erste Datenbank zur Ermittlung der vorhandenen Fähigkeiten bzw. Fähigkeitsdefizite ausgewertet, die für die zu behandelnde Fertigkeit relevant sind. Auf Basis dieser Auswertung werden von der Datenverarbeitungsstation durch Rückgriff auf die zweite Datenbank unter Berücksichtigung der Expertenregeln Übungen und/oder zu behandelnde Fähigkeiten ausgewählt und mit einer Information über die Gewichtung und/oder Reihenfolge bei der Durchführung eines Trainings, vorzugsweise zusammen mit der jeweils zu behandelnden Fertigkeit, ausgegeben.

Die jeweils zu behandelnde Fertigkeit kann bspw. von dem Benutzer eingegeben werden. Vorzugsweise werden die ein oder mehreren zu behandelnden Fertigkeiten von der Datenverarbeitungsstation jedoch automatisch aus einem Fertigkeitsprofil des Patienten ermittelt, das für die Durchführung des Verfahrens bereitgestellt wird. Die Ermittlung der behandlungsbedürftigen Fertigkeiten erfolgt dabei über den im Fertigkeitsprofil angegebenen Grad oder das Defizit der jeweiligen Fertigkeit. Liegt dieser Grad der Fertigkeit unterhalb bzw. das Defizit oberhalb eines vorgebbaren Wertes relativ zur 100%igen Fertigkeit einer Vergleichsperson, so wird diese Fertigkeit als behandlungsbedürftig eingestuft. Das Fähigkeits- sowie das Fertigkeitsprofil des Patienten werden vorzugsweise über eine Datenbank bereitgestellt, aus der sie von der Datenverarbeitungsstation abgerufen werden. Selbstverständlich können hierbei sowohl unterschiedliche Datenbanken für das Fertigkeitsprofil und das Fähigkeitsprofil als auch eine gemeinsame Datenbank für beide Profile eingesetzt werden.

Durch die automatische Auswertung des Fähigkeitsprofils sowie ggf. des Fertigkeitsprofils und die automatische Auswahl von Übungen sowie deren Gewichtung und/oder Reihenfolge durch die Datenverarbeitungsstation erhält der Benutzer, d. h. der Arzt oder Therapeut, einen Vorschlag für die Erstellung eines Trainingsprogrammes, das bereits individuell an die Ausgangssituation des Patienten angepasst ist. Die Expertenregeln in der zweiten Datenbank sind dabei so ausgelegt, dass eine schnellstmögliche Erlangung der jeweiligen Fertigkeiten mit dem Trainingsprogramm erreicht wird. Das dem Arzt vorgeschlagene Trainingsprogramm in Form von ein oder mehreren Übungen sowie deren Reihenfolge und/oder Gewichtung, stellt somit sicher, dass der Patient in kürzest möglichem Zeitraum die entsprechenden Fertigkeiten wieder erlangt. Ein schneller Behandlungserfolg wiederum hebt die Lebenszuversicht und Compliance des Patienten. Insgesamt wird somit unter Verwendung des vorgeschlagenen Verfahrens und Systems die Lebensqualität des Patienten mit den verfügbaren Therapieoptionen in kürzest möglicher Zeit so gut wie möglich gesteigert. Selbstverständlich hat der Benutzer noch die Möglichkeit, das von der Datenverarbeitungsstation vorgeschlagene Trainingsprogramm zu modifizieren, falls er dies aufgrund weitergehender Erfahrungen für erforderlich hält.

Die in der zweiten Datenbank enthaltenen Expertenregeln können relativ einfach gestaltet sein, bspw. indem der Schwierigkeitsgrad der Übungen dem jeweiligen Grad des Defizits angepasst ist. Weiterhin kann der angegebene Zeitaufwand für jede ausgewählte Übung entsprechend dem prozentualen Defizit der zugehörigen Fähigkeiten gewichtet gewählt sein. Liegen bspw. zwei Defizite mit je 80% und 20% vor, dann kann die Dauer der zugehörigen Übungen durch die Regel ebenfalls mit 80% und 20% gewählt werden. Vorzugsweise sind die Regeln jedoch komplexer gestaltet und beinhalten Erfahrungen von Ärzten oder allgemeine medizinische Erfahrung bei der Behandlung von Fertigkeiten. Insbesondere sollten die Regeln das Maß der jeweils individuell vorliegenden Defizite berücksichtigen.

In einer weiteren Ausgestaltung des vorliegenden Verfahrens wird eine weitere Datenbank bereitgestellt, die mehrere Fertigkeiten sowie eine Priorisierung der Fertigkeiten enthält. Diese Priorisierung gibt an, in welcher Reihenfolge die jeweiligen Fertigkeitsdefizite zu behandeln sind. Die Priorisierung kann hierbei unter Berücksichtigung der medizinischen Notwendigkeit, von einschränkenden Randbedingungen, die in den persönlichen Umständen des Patienten begründet sind, oder von persönlichen Präferenzen des Patienten erstellt sein. Diese Priorisierung kann im ersten Schritt automatisch erfolgen, indem sowohl medizinisch anerkannte Kriterien als auch weitere Kriterien, die für den üblichen Durchschnitt der Bevölkerung einer Region gelten, herangezogen werden. Ein medizinisches Kriterien ist bspw. die Lebensnotwendigkeit einer Fertigkeit wie z. B. die Atmung ohne technische Hilfsmittel, das Schlucken usw.. In nächster Prioritätsstufe kann das Erreichen einer lebensnotwendigen Fertigkeit ohne Hilfe Dritter zu sehen sein, wie bspw. selbständiges Essen. In weiteren Prioritätsstufen können dann zwingend notwendige Aufgaben des täglichen Lebens, wie Sprechen oder andere Kommunikationsmittel, Gang zur Toilette, Anziehen, Waschen, Essenszubereitung, usw. vorgesehen sein. Schließlich können als weitere Kategorien auch notwendige Aufgaben, bei welchen eine Abhängigkeit von Dritten die Lebensqualität deutlich einschränkt, wie bspw. Einkaufen, Geld abheben, Treppe steigen, oder Tätigkeiten, die wesentlicher Bestandteil der Lebensqualität sind, wie bspw. Auto fahren, Telefonieren, ein Vollbad nehmen, die Bedienung technischer Geräte usw., bei der Priorisierung berücksichtigt werden. Insbesondere in den letzten beiden Kategorien der Priorisierung sind Standards aus medizinischer Erfahrung oder über einen Bevölkerungsdurchschnitt hinweg hilfreich. Hier können Vorlieben und Wünsche des Patienten einfließen, welche der behandelnde Arzt oder Therapeut durch Befragen ermitteln kann. Darüber hinaus wird der Arzt noch Prioritäten dadurch verändern wollen, dass vorliegende Randbedingungen es z. B. wenig aussichtsreich erscheinen lassen, dass eine Fertigkeit höherer Priorität wieder erreicht wird, während eine andere niedrigerer Priorität mit guter Aussicht in überschaubarem Zeitraum erreichbar ist. Derartige Randbedingungen können bspw. das Alter, die Intelligenz, die körperliche Konstitution usw. des Patienten sein. Der Arzt wird dann vermutlich der zweiten Fertigkeit eine individuell höhere Priorität im Behandlungsplan des Patienten zuordnen. Aus diesem Grund ist nach einer automatisierten oder durch die Datenbank vorgegebenen Priorisierung der Reihenfolge der zu behandelnde Fertigkeitsdefizite die Möglichkeit vorhanden, die Priorisierung bzw. Prioritätsliste interaktiv zu verändern. Die Datenverarbeitungsstation wählt bei dieser Ausgestaltung der Erfindung die Fertigkeit mit der höchsten Priorität aus und gibt entsprechende Übungen sowie deren Gewichtung und/oder Reihenfolge aus, die für die Behandlung der entsprechenden Fertigkeit geeignet sind.

In einer besonders bevorzugten Ausführungsform des vorliegenden Verfahrens sowie des zugehörigen Systems wird im Verlauf der Therapie wiederholt ein aktuelles Fähigkeits- und ggf. Fertigkeitsprofil des Patienten bereitgestellt, um bei einer Änderung einzelner Fähigkeiten bzw. Fähigkeitsdefizite durch erneuten Rückgriff auf die Expertenregeln der entsprechenden Datenbank automatisch Vorschläge zur Modifikation des Trainingsprogrammes zu generieren. Die Erhebung des jeweils aktuellen Fähigkeitsprofils wird vorzugsweise in regelmäßigen Abständen durchgeführt und der entsprechende Datenbankinhalt geändert. Eine solche Erhebung kann bei telemedizinischen Betreuungsformen ggf. auch durch Datenfernübertragung vom Heim des Patienten in die Klinik erfolgen, bspw. durch Messung des Übungserfolges computerbasierter Trainingsprogramme oder durch computerbasierte Erhebung von Fragebogen zur Lebensqualität. Die hierbei mögliche automatische Erhebung des Fähigkeitsprofils erfolgt über den im heimischen Umfeld des Patienten zur Verfügung gestellten Trainingscomputer. Bei Vorliegen veränderter Fähigkeitsprofile wird die bereits beschriebene Auswertung sowie die Ausgabe von Übungen mit deren Gewichtung und/oder Reihenfolge durch Rückgriff auf die Expertenregeln erneut vorgenommen. Auf diese Weise kann das verschriebene Trainingsprogramm ständig angepasst werden, immer mit dem Ziel der Optimierung hinsichtlich der Behandlung von individuell priorisierten Fertigkeitsdefiziten. Insbesondere ist es von Vorteil, bei mehreren gleichzeitig behandelten Fähigkeitsdefiziten den Behandlungsfortschritt jeder Fähigkeit, d. h. die Verminderung des jeweiligen prozentualen Defizits, zu messen und zwischen den verschiedenen Fähigkeiten zu vergleichen. Ziel des Vergleichs und des automatisch daraus abgeleiteten Vorschlags für eine Modifikation des Trainingsprogrammes ist es, einen ausgewogenen Therapiefortschritt in allen Fähigkeiten zu erreichen. Dies ist aus mehreren Gründen ein besonders sinnvolles Verfahren zur Qualitätskontrolle der Therapie. So ist zum einen das eigentliche Ziel die Wiedererlangung einer bestimmten Fertigkeit. Für diese wird das Wiedererlangen aller gleichzeitig behandelten Fähigkeiten benötigt. Somit ist es für den Behandlungserfolg nicht hilfreich, wenn manche Fähigkeiten einen sehr guten Fortschritt erzielen, andere aber deutlich zurückbleiben, da dann das eigentliche Ziel, die Wiedererlangung der Fertigkeit, nicht erreicht wird. Daher muss das Trainingsprogramm in diesem Fall zugunsten der Fähigkeiten, die eine geringere Verbesserung zeigen, verschoben werden. Der eben beschriebene Effekt wird häufig noch dadurch verstärkt, dass Übungserfolge den Patienten gerade bei den Fähigkeiten verstärkt motivieren, die ohnehin weniger kritisch sind. Ein weiterer bekannter Effekt eines Übertrainings ist das Auswendiglernen, d. h. die reflexartige Ausführung der Übungsaufgabe im Übungskontext, ohne dass die gleiche Aufgabe in einem anderen Kontext, z. B. beim Ausführen der Ziel-Fertigkeit, genauso gut abrufbar ist. Durch die ständige Neuerhebung des Fähigkeits- und ggf. Fertigkeitsprofils und die entsprechende Auswertung und Neubewertung der Situation wird das Trainingsprogramm jeweils an die aktuelle Situation angepasst, um ein schnellstmögliches Erlangen der Zielfertigkeit zu gewährleisten.

Mit diesem Verfahren gemäß der vorangehend erläuterten Ausführungsform wird eine automatisierte Überwachung des Therapieverlaufs und eine automatisierte Erstellung von Vorschlägen für eine Modifikation des Trainingsprogrammes zur Verfügung gestellt, bei der ein ausgewogener Fortschritt aller relevanten Fähigkeiten mit dem Ziel der Wiedererlangung einer Fertigkeit gewährleistet wird. Das vorgeschlagene Verfahren bzw. System erkennt somit durch wiederholte Auswertung der Fähigkeitsprofile einen unausgewogenen Therapiefortschritt bei der gleichzeitigen Therapie mehrerer Fähigkeitsdefizite und erstellt automatisch einen Vorschlag für eine geeignete Modifikation des Trainingsprogrammes.

Das zugehörige System zur Unterstützung der Therapieplanung umfasst eine Datenverarbeitungsstation, die mit der ersten und zweiten Datenbank verbunden ist. In der Datenverarbeitungsstation ist ein Modul zur automatischen Auswertung eines Fähigkeitsprofils eines Patienten durch Rückgriff auf die erste Datenbank zur Ermittlung der vorhandenen Fähigkeiten bzw. Fähigkeitsdefizite, die für eine zu behandelnde Fertigkeit relevant sind, und zur Auswahl und Ausgabe von Übungen und/oder zu behandelnden Fähigkeiten mit einer Information über die Gewichtung und/oder Reihenfolge bei der Durchführung eines Trainings der zu behandelnden Fertigkeit durch Rückgriff auf die zweite Datenbank und Berücksichtigung der Expertenregeln.

In den weiteren Ausgestaltungen des vorliegenden Systems ist die Datenverarbeitungsstation mit einer oder mehreren der weiteren Datenbanken verbunden und das Modul entsprechend zur Durchführung der vorangehend erläuterten Verfahrensschritte ausgebildet.

Das vorliegende Verfahren und das zugehörige System werden nachfolgend anhand eines Ausführungsbeispiels in Verbindung mit den Zeichnungen ohne Beschränkung des allgemeinen Erfindungsgedankens nochmals erläutert. Hierbei zeigen:
- Fig. 1: ein Beispiel für ein Fähigkeitsprofil eines Patienten (auszugsweise);
- Fig. 2: ein Beispiel für ein Fertigkeitsprofil eines Patienten (auszugsweise);
- Fig. 3: ein Beispiel für eine erste Datenbank, die mehrere Fertigkeiten und eine Zuordnung von Mindestvoraussetzungen an Fähigkeiten enthält, die für die jeweilige Fertigkeit erforderlich sind;
- Fig. 4: ein Beispiel für eine Datenbank mit Therapiemodulen bzw. Übungen und zugeordneten ZielFähigkeiten, die mit der jeweiligen Übung trainiert werden; und

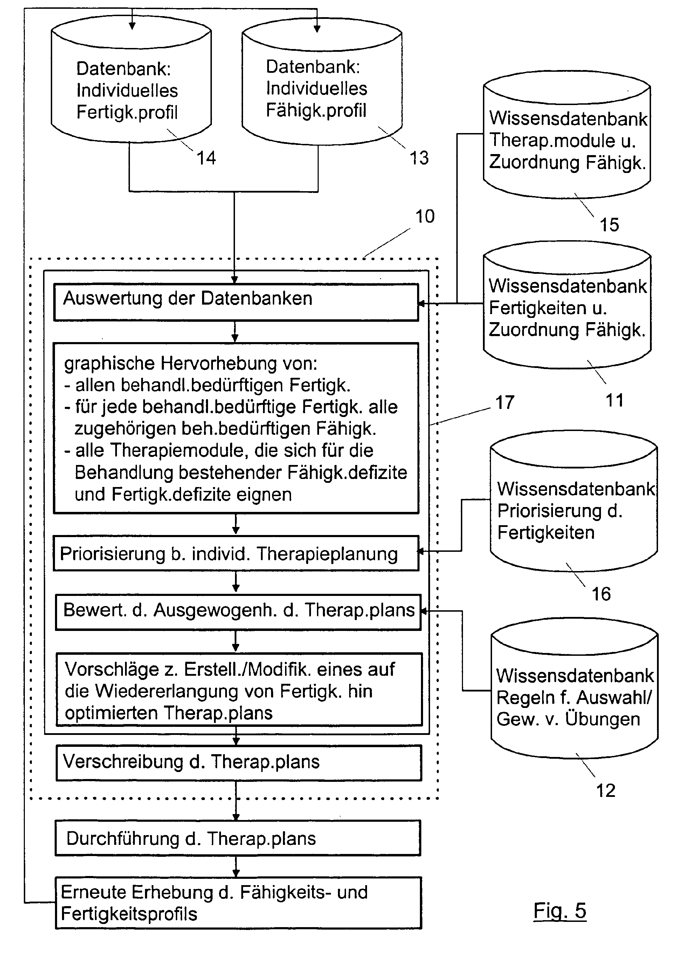

- Fig. 5: einen Überblick über das vorliegende Verfahren sowie das zugehörige System in einer Ausgestaltung der Erfindung.

Im vorliegenden Ausführungsbeispiel wird der Vorgang der Erstellung eines Trainingsprogrammes in der Therapieplanung durch einen Arzt oder Therapeuten unter Einsatz des vorliegenden Verfahrens sowie des zugehörigen Systems in einer speziellen Ausgestaltung beispielhaft erläutert. Das beispielhafte System umfasst einen Computerarbeitsplatz (Datenverarbeitungsstation 10) zur Therapieplanung und Therapieverlaufskontrolle mit einem Modul 17 zur automatisierten Auswertung der durch Datenbanken zur Verfügung gestellten Daten. Die Datenverarbeitungsstation 10 ist mit den verschiedenen Datenbanken verbunden, aus der das Modul 17 die erforderlichen Informationen abruft. In der Grundausstattung dieses beispielhaften Systems sind eine erste Wissens-Datenbank 11 mit Fertigkeiten und einer Zuordnung von erforderlichen Fähigkeiten, eine Wissensdatenbank 12 mit Expertenregeln für die Auswahl von Übungen und deren Reihenfolge und/oder Gewichtung zur Behandlung unterschiedlicher Fertigkeiten sowie eine Datenbank 13 mit dem individuellen Fähigkeitsprofil des Patienten vorgesehen.

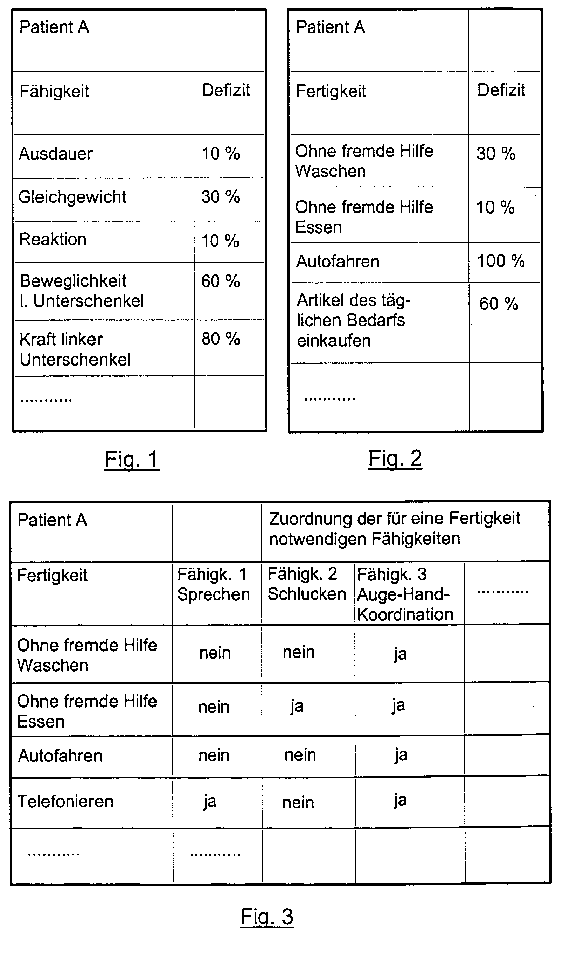

Ein Beispiel für ein Fähigkeitsprofil wie es in der entsprechenden Datenbank 13 enthalten ist, zeigt Figur 1 im Auszug. Dieses Fähigkeitsprofil umfasst unterschiedliche Fähigkeiten, wie Ausdauer, Gleichgewicht usw. mit dem jeweiligen Defizit, d. h. dem Prozentsatz, zu dem bei diesem Patienten die jeweilige Fähigkeit bezüglich der 100%igen Fähigkeit einer gesunden Vergleichsperson verringert ist.

Die erste Wissens-Datenbank 11 enthält eine Vielzahl von Fertigkeiten sowie den jeweiligen Fertigkeiten zugeordnete Mindestvoraussetzungen an Fähigkeiten, die für die Fertigkeit erforderlich sind. Ein Beispiel für eine derartige Zuordnung ist in Figur 3 dargestellt. Aus dieser Darstellung ist ersichtlich, das bspw. zur Erlangung der Fertigkeit des Essens ohne fremde Hilfe die Fähigkeit des Sprechens nicht erforderlich ist, während die Fähigkeit des Schluckens sowie der Auge-Hand-Koordination notwendige Voraussetzungen sind. Die letztgenannten Fähigkeiten müssen daher trainiert werden, falls sie beim Patienten nicht in ausreichendem Maße vorhanden sind. Zusätzlich ist in dieser Datenbank zu jeder Fähigkeit noch eine Angabe des Grades enthalten, zu dem die Fähigkeit für die jeweilige Fertigkeit vorliegen muss, falls dieser Grad unterhalb von 100% liegt. Dies ist aus der Figur 3 nicht ersichtlich.

Optional können beim vorliegenden System weitere Datenbanken mit der Datenverarbeitungsstation 10 verbunden sein. So kann eine Datenbank 14 mit einem individuellen Fertigkeitsprofil des Patienten vorgesehen sein. Der Inhalt dieser Datenbank umfasst die einzelnen Fertigkeiten sowie das zugehörige Defizit, wie dies beispielhaft anhand der Figur 2 ausschnittsweise dargestellt ist. Im Modul 17 der Datenverarbeitungsstation 10 wird durch Zugriff auf diese Datenbank 14 das Fertigkeitsprofil abgerufen und ausgewertet, um die zu behandelnden Fertigkeiten, bei denen das Defizit mehr als bspw. 0% beträgt, zu ermitteln. Auf Basis der ermittelten, zu behandelnden Fertigkeiten wird dann von dem Modul 17 durch Rückgriff auf die weiteren in jedem Fall zur Verfügung stehenden Datenbanken 11, 12 automatisch ein Vorschlag für ein Trainingsprogramm, d. h. die einzelnen Übungen, den Schwierigkeitsgrad der Übungen sowie die Reihenfolge und/oder Gewichtung, insbesondere die Dauer der einzelnen Übungen, erstellt und an einem Monitor für den Benutzer ausgegeben. Die Ausgabe kann selbstverständlich auch in anderer Weise, bspw. über einen Drucker erfolgen.

Optional kann bei dem vorliegenden System auch eine dritte Wissensdatenbank 16 vorgesehen sein, die eine Priorisierung der behandlungsbedürftigen Fertigkeiten enthält. Das Modul 17 gibt dann entweder nur ein Trainingsprogramm für die Fertigkeit mit der höchsten Priorität oder mehrere Trainingsprogramme in der Reihenfolge der Prioritäten der zu behandelnden Fertigkeiten aus.

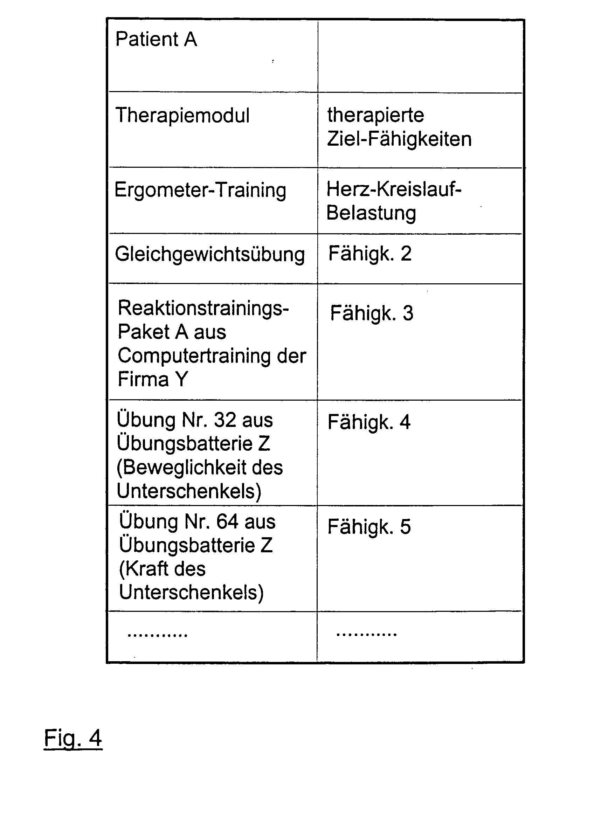

Zusätzlich kann eine vierte Wissensdatenbank 15 vorgesehen sein, die mehrere Therapiemodule bzw. Übungen sowie eine Zuordnung von Ziel-Fähigkeiten enthält, die mit dem jeweiligen Therapiemodul behandelt werden. Ein Beispiel für eine derartige Zuordnung ist in der Figur 4 dargestellt.

Bei der Benutzung des vorliegenden Verfahrens bzw. des zugehörigen Systems hat der Arzt oder Therapeut bei der Therapieplanung am Computerarbeitsplatz 10 Zugriff auf alle Therapiemodule bzw. Übungen, die ihm zur Verordnung zur Verfügung stehen. Durch automatische Auswertung der mit der Datenverarbeitungsstation 10 verbundenen Datenbanken, insbesondere der Datenbank 13 mit dem Fähigkeitsprofil, der ersten und zweiten Wissensdatenbanken 11, 12 sowie ggf. der Datenbank 13 mit dem Fertigkeitsprofil, können in einem ersten Schritt optional alle behandlungsbedürftigen Fertigkeiten, für jede behandlungsbedürftige Fertigkeit alle zugehörigen behandlungsbedürftigen Fähigkeiten sowie - bei Rückgriff auf die vierte Wissensdatenbank 15 - alle Therapiemodule ausgegeben bzw. angezeigt werden, die sich für die Behandlung bestehender Fähigkeitsdefizite und Fertigkeitsdefizite eignen. Die entsprechenden Fertigkeiten und Fähigkeiten können zusätzlich graphisch hervorgehoben werden. Diese Anzeige bzw. Ausgabe ist jedoch beim vorliegenden Verfahren nicht in jedem Falle erforderlich. Vielmehr kann durch Rückgriff auf die erste und zweite Wissensdatenbank 11, 12 durch das Modul 17 automatisch ein Vorschlag für Übungen sowie deren Reihenfolge und/oder Gewichtung ausgegeben werden, der auf Basis der Expertenregeln zu einem optimalen, insbesondere ausgewogenen Trainingsprogramm führt. Bei Vorliegen mehrerer behandlungsbedürftiger Fertigkeitsdefizite kann durch Rückgriff auf die dritte Wissensdatenbank 16 mit der Priorisierung zunächst lediglich der Vorschlag für die zu behandelnde Fertigkeit mit der höchsten Priorisierung oder auch mehrere Vorschläge in der Reihenfolge der Priorisierung ausgegeben werden.

Auf Basis dieser Ausgabe der Datenverarbeitungsstation 10, in der bereits die Regeln für eine möglichst schnelle Erlangung der zu behandelnden Fertigkeiten berücksichtigt sind, kann der Arzt oder Therapeut ohne großen Zeitaufwand ein individuell an den Patienten angepasstes Trainingsprogramm verordnen. Der Arzt oder Therapeut kann hierbei das vorgeschlagene Trainingsprogramm identisch übernehmen oder ggf. am Computerarbeitsplatz geeignet anpassen bzw. verändern.

Nach der Verordnung eines Trainingsprogrammes wird bei diesem Ausführungsbeispiel wiederholt während der Durchführung des Trainingsprogrammes erneut das Fähigkeits- und ggf. das Fertigkeitsprofil des Patienten erhoben und in den jeweiligen Datenbanken 13, 14 abgespeichert. Das Modul 17 des Computerarbeitsplatzes 10 prüft in diesem Fall die entsprechenden Profile auf Veränderungen und wiederholt die für die ursprüngliche Erstellung des Trainingsprogrammes durchgeführten Schritte, so dass ggf. ein angepasstes bzw. verändertes Trainingsprogramm ausgegeben wird. Dieses angepasste Trainingsprogramm kann der Arzt oder Therapeut dann dem weiteren Training zugrunde legen. Durch diese wiederholte Therapiekontrolle wird automatisch unmittelbar auf Veränderungen reagiert, die eine Modifikation des Trainingsprogrammes erforderlich machen, und ein entsprechend modifiziertes Trainingsprogramm ausgegeben. Dies ermöglicht eine Optimierung der Therapie, um eine schnellstmögliche Erlangung der Fertigkeiten zu erreichen.

Durch das beispielhaft dargestellte Verfahren und das zugehörige System werden durch Nutzung von Datenbanken und graphischen Bedienungsoberflächen an einem Computerarbeitsplatz dem behandelnden Arzt oder Therapeuten automatisch Vorschläge für die Erstellung eines Trainingsprogrammes bereitgestellt, die eine Optimierung der Therapieplanung im Hinblick auf individuelle Fertigkeitsdefizite ermöglichen, und insbesondere eine Ausgewogenheit der Therapie zugehöriger Fähigkeitsdefizite gewährleistet.

## Patentansprüche

1. Verfahren zur Unterstützung der Therapieplanung bei der Erstellung eines Trainingsprogrammes, bei dem ein Fähigkeitsprofil eines Patienten, eine erste Datenbank (11), die mehrere Fertigkeiten und eine Zuordnung von Mindestvoraussetzungen an Fähigkeiten enthält, die für die jeweilige Fertigkeit erforderlich sind, und eine zweite Datenbank (12) bereitgestellt werden, die mehrere Fertigkeiten mit Expertenregeln über die Auswahl von Übungen und/oder zu behandelnden Fähigkeiten sowie deren Reihenfolge und/oder Gewichtung zur Erlangung der jeweiligen Fertigkeiten unter Berücksichtigung vorhandener Fähigkeiten bzw. Fähigkeitsdefizite enthält, und von einer Datenverarbeitungsstation (10) für zumindest eine zu behandelnde Fertigkeit des Patienten durch Rückgriff auf die erste Datenbank (11) automatisch das Fähigkeitsprofil des Patienten zur Ermittlung der vorhandenen Fähigkeiten bzw. Fähigkeitsdefizite ausgewertet wird und durch Rückgriff auf die zweite Datenbank (12) unter Berücksichtigung der Expertenregeln Übungen und/oder zu behandelnde Fähigkeiten ausgewählt und mit einer Information über die Gewichtung und/oder Reihenfolge bei der Durchführung eines Trainings ausgegeben werden.

2. Verfahren nach Anspruch 1,
**dadurch gekennzeichnet,**
**dass** ein Fertigkeitsprofil des Patienten bereitgestellt wird, aus dem die Datenverarbeitungsstation (10) die zu behandelnden Fertigkeiten automatisch ermittelt.

3. Verfahren nach Anspruch 1 oder 2,
**dadurch gekennzeichnet,**
**dass** das Fähigkeits- und/oder Fertigkeitsprofil des Patienten von einer dritten (13) und/oder vierten Datenbank (14) abgerufen wird.

4. Verfahren nach einem der Ansprüche 1 bis 3,
**dadurch gekennzeichnet,**
**dass** von der Datenverarbeitungsstation (10) automatisch für jede ausgegebene Übung ein oder mehrere zugeordnete Ziel-Fähigkeiten ausgegeben werden.

5. Verfahren nach Anspruch 4,
**dadurch gekennzeichnet,**
**dass** die zugeordneten Ziel-Fähigkeiten von einer fünften Datenbank (15) abgerufen werden, die mehrere Übungen und eine Zuordnung von Ziel-Fähigkeiten, die bei der Durchführung der jeweiligen Übung trainiert werden, enthält.

6. Verfahren nach einem der Ansprüche 1 bis 5,
**dadurch gekennzeichnet,**
**dass** eine sechste Datenbank (16) bereitgestellt wird, die mehrere Fertigkeiten sowie eine Priorisierung der Fertigkeiten enthält.

7. Verfahren nach Anspruch 6,
**dadurch gekennzeichnet,**
**dass** die Priorisierung der Fertigkeiten in der sechsten Datenbank (16) durch einen Benutzer verändert werden kann.

8. Verfahren nach Anspruch 6 oder 7,
**dadurch gekennzeichnet,**
**dass** die Datenverarbeitungsstation (10) durch Rückgriff auf die sechste Datenbank (16) automatisch Übungen auswählt, mit denen behandlungsbedürftige Fähigkeiten, die zu der zu behandelnden Fertigkeit mit der höchsten Priorisierung gehören, behandelbar sind.

9. Verfahren nach einem der Ansprüche 1 bis 8,
**dadurch gekennzeichnet,**
**dass** die Expertenregeln in der zweiten Datenbank (12) über die Auswahl von Übungen und/oder zu behandelnden Fähigkeiten sowie deren Reihenfolge und/oder Gewichtung zur schnellstmöglichen Erlangung der jeweiligen Fertigkeiten ausgelegt sind.

10. Verfahren nach einem der Ansprüche 1 bis 9,
**dadurch gekennzeichnet,**
**dass** von der Datenverarbeitungsstation (10) automatisch für alle Übungen und/oder zu behandelnden Fähigkeiten eine zugehörige Organisationseinheit und/oder Organisationskategorie ausgegeben wird, die für die Durchführung der Übung bzw. Behandlung der Fähigkeit zuständig ist.

11. Verfahren nach einem der Ansprüche 1 bis 10,
**dadurch gekennzeichnet,**
**dass** im Verlauf einer Therapie wiederholt ein aktuelles Fähigkeitsprofil des Patienten bereitgestellt wird, um bei einer Änderung einzelner Fähigkeiten durch erneuten Rückgriff auf die Expertenregeln der zweiten Datenbank (12) automatisch Vorschläge zur Modifikation des Trainingsprogramms zu generieren.

12. System zur Unterstützung der Therapieplanung bei der Erstellung eines Trainingsprogrammes mit einer Datenverarbeitungsstation (10), die mit einer ersten Datenbank (11), die mehrere Fertigkeiten und eine Zuordnung von Mindestvoraussetzungen an Fähigkeiten enthält, die für die jeweilige Fertigkeit erforderlich sind, und mit einer zweiten Datenbank (12) verbunden ist, die mehrere Fertigkeiten mit Expertenregeln über die Auswahl von Übungen und/oder zu behandelnden Fähigkeiten sowie deren Reihenfolge und/oder Gewichtung zur Erlangung der jeweiligen Fertigkeiten unter Berücksichtigung vorhandener Fähigkeiten bzw. Fähigkeitsdefizite enthält, und ein Modul (17) zur automatischen Auswertung eines Fähigkeitsprofils eines Patienten durch Rückgriff auf die erste Datenbank (11) zur Ermittlung der für eine zu behandelnde Fertigkeit vorhandenen Fähigkeiten bzw. Fähigkeitsdefizite und zur Auswahl und Ausgabe von Übungen und/oder zu behandelnden Fähigkeiten mit einer Information über die Gewichtung und/oder Reihenfolge bei der Durchführung eines Trainings durch Rückgriff auf die zweite Datenbank (12) und Berücksichtigung der Expertenregeln.

13. System nach Anspruch 12,
**dadurch gekennzeichnet,**
**dass** das Modul (17) zur automatischen Ermittlung der zu behandelnden Fertigkeiten des Patienten auf der Grundlage eines Fertigkeitsprofils des Patienten ausgebildet ist.

14. System nach Anspruch 12 oder 13,
**dadurch gekennzeichnet,**
**dass** die Datenverarbeitungsstation (10) mit einer dritten (13) und/oder vierten Datenbank (14) verbunden ist, aus denen das Fähigkeits- und/oder Fertigkeitsprofil abrufbar ist.

15. System nach einem der Ansprüche 12 bis 14,
**dadurch gekennzeichnet,**
**dass** die Datenverarbeitungsstation mit einer sechsten Datenbank (16) verbunden ist, die mehrere Fertigkeiten sowie eine Priorisierung der Fertigkeiten enthält, und das Modul (17) zur automatischen Auswahl von Übungen durch Rückgriff auf die sechste Datenbank (16) ausgebildet ist, mit denen behandlungsbedürftige Fähigkeiten, die zu der zu behandelnden Fertigkeit mit der höchsten Priorisierung gehören, behandelbar sind.

16. Verfahren nach Anspruch 15,
**dadurch gekennzeichnet,**
**dass** das Modul (17) eine Veränderung der Priorisierung durch den Benutzer ermöglicht.

17. System nach einem der Ansprüche 12 bis 16,
**dadurch gekennzeichnet,**
**dass** das Modul (17) zum wiederholten Abruf des Fähigkeitsprofils des Patienten im Verlauf der Therapie ausgebildet ist, um bei einer Änderung einzelner Fähigkeiten durch erneuten Rückgriff auf die Expertenregeln der zweiten Datenbank (12) automatisch Vorschläge zur Modifikation des Trainingsprogramms zu generieren.

18. System nach einem der Ansprüche 12 bis 17,
**dadurch gekennzeichnet,**
**dass** die Expertenregeln in der zweiten Datenbank (12) zur schnellstmöglichen Erlangung der jeweiligen Fertigkeiten angelegt sind.
